# EUROPEAN PATENT SPECIFICATION

(11) **EP 4 085 861 B1**
(45) Date of publication and mention of the grant of the patent: **02.09.2026**
(21) Application number: 20910007.2
(22) Date of filing: 29.09.2020
(51) Int. Cl.: A61B 18/14

(54) **RADIO-FREQUENCY ABLATION CATHETER AND RADIO-FREQUENCY ABLATION SYSTEM**
HOCHFREQUENZ-ABLATIONSKATHETER UND HOCHFREQUENZ-ABLATIONSSYSTEM
CATHÉTER D'ABLATION PAR RADIOFRÉQUENCE ET SYSTÈME D'ABLATION PAR RADIOFRÉQUENCE

(30) Priority: 31.12.2019 CN 201911403420
(43) Date of publication of application: 09.11.2022
(73) Proprietor: Hangzhou Broncus Medical Co., Ltd., Hangzhou, Zhejiang 310051 (CN)
(72) Inventor: WANG, Liming, Hangzhou, Zhejiang 310051 (CN); XU, Hong, Hangzhou, Zhejiang 310051 (CN); ZHOU, Huazhen, Hangzhou, Zhejiang 310051 (CN)
(74) Representative: MERH-IP Matias Erny Reichl Hoffmann Patentanwälte PartG mbB
(86) International application number: PCT/CN2020/118645
(87) International publication number: WO 2021/135460

(56) References cited:
- WO-A1-01/70114
- CN-A- 101 888 874
- CN-A- 105 434 040
- CN-A- 106 308 927
- CN-A- 111 012 481
- CN-A- 111 166 465
- CN-A- 111 166 466
- CN-Y- 2 925 405
- US-A1- 2002 111 615
- US-A1- 2007 112 342
- US-A1- 2008 039 793
- US-A1- 2011 125 147
- US-A1- 2011 125 147
- US-A1- 2018 049 802
- US-A1- 2019 209 229

## Description

### TECHNICAL FIELD

Embodiments of the present disclosure relate to the field of medical instruments, and particularly to a radio-frequency ablation catheter and a radio-frequency ablation system.

### BACKGROUND

The radio-frequency ablation technology is widely applied in a lung surgery. Radio-frequency refers to radio frequency, but does not belong to division of bands in radio communication. A main effect on organisms is a thermal effect. When a current frequency of the radio frequency reaches a certain value (>100kHz), charged ions within a tissue will move, that is, heat (60°C to 100°C) is generated by friction. A frequency commonly used by a radio-frequency ablation device ranges 200kHz to 500kHz and an output power thereof ranges from 100kHz to 400kHz. An ablation electrode is a core component of a radio-frequency ablation system, because it directly affects a size and a shape of coagulation necrosis. An ideal shape of a coagulation zone should be spherical or oblate. Under the guidance of B-ultrasound or CT, a multineedle electrode is directly punctured into a pathological tissue mass. A radio-frequency electrode needle may make a temperature within the tissue exceed 60°C to cause cell death and produce a necrosis zone. If a local temperature of the tissue exceeds 100°C, the coagulation necrosis will occur in a tumor tissue and parenchyma surrounding the organ, and a large spherical coagulation necrosis zone may be produced during treatment. A hyperthermia zone of 43°C to 60°C exists outside the coagulation necrosis zone. In this zone, cancer cells may be killed and normal cells may be restored.

In a treatment process, the radio-frequency electrode is extended into a human tissue, and a current enters a lesion through the radio-frequency electrode, resulting in a large amount of heat at the radio-frequency electrode. For example, when a temperature at the lesion reaches 40°C to 60°C and remains for a period of time, the ablation surgery at the lesion is completed. However, a radio-frequency ablation system in the prior art may not determine working state information of the radio-frequency electrode, such as a temperature near the radio-frequency electrode. Therefore, in a surgical process, the progress of the ablation surgery may be determined and adjusted only based on doctor's experiences, which increases the surgical difficulty and accuracy. Accordingly, how to provide a radio-frequency ablation system such that it may accurately determine whether ablation is completed is an urgent problem to be
solved in the art. A tissue ablation apparatus and method for controlling the extent of tissue ablation are known from U.S. Patent application publication US 2007 / 112 342 A1, which utilizes delivering energy from an energy deliver device to a plurality of deployed electrodes and regulating energy delivered to the electrodes based on the temperature sensed by at least one remote elongate sensor element. A lung treatment apparatus known from International Publication Number WO 01 / 70114 A1 includes an elongated member having a proximal portion, a distal portion and a lumen. An energy delivery device is coupled to the distal portion of the elongated member.

### SUMMARY

The invention is set out in the appended set of claims. Preferable embodiments are defined in the dependent claims. In particular, an objective of the present disclosure is to provide a radio-frequency ablation catheter applied to a radio-frequency ablation system. A plurality of temperature sensors on a signal conduit are configured to detect temperatures near a plurality of sub-needles and transmit a temperature change range to the radio-frequency ablation system, so as to obtain a specific range of a local temperature of a tissue in a surgical process.

Embodiments of the present disclosure provide a radio-frequency ablation catheter applied to a radio-frequency ablation system, including a needle tube portion and a handle portion, wherein
the handle portion includes a sleeve and a booster, wherein the sleeve is sleeved on the booster, the booster is slidably arranged at one end of the sleeve, the booster is provided with a conductive joint, and the conductive joint is used for an external radio-frequency ablation system;
the needle tube portion includes a puncture tube, an electrode tube and a signal conduit, wherein
the puncture tube is fixed at the other end of the sleeve, the electrode tube is slidably disposed within the puncture tube, one end of the electrode tube is fixed on the conductive joint and the other end of the electrode tube is provided with a plurality of sub-needles, the plurality of sub-needles are configured to transmit a current provided by the conductive joint, the signal conduit is slidably disposed in the puncture tube, the signal conduit is positioned on one side of the electrode tube, and one end of the signal conduit is fixed on the conductive joint;
the signal conduit includes a plurality of supports and a plurality of temperature sensors;
the other end of the signal conduit is provided with the plurality of supports, and the plurality of supports are positioned on one sides of the plurality of sub-needles; and
the plurality of temperature sensors are disposed on the plurality of supports, the temperature sensors are electrically conducted to the plurality of supports, and the plurality of temperature sensors are configured to detect temperatures near the plurality of sub-needles and transmit them to the radio-frequency ablation system through the signal conduit.

In a feasible solution, the plurality of temperature sensors of the radio-frequency ablation catheter are positioned at ends of the plurality of supports.

In a feasible solution, the temperature sensors of the radio-frequency ablation catheter are capacitance thermometers.

In a feasible solution, the plurality of supports and the plurality of sub-needles of the radio-frequency ablation catheter are identical in numbers and are arranged alternately.

In a feasible solution, the plurality of supports are disposed next to the plurality of sub-needles of the radio-frequency ablation catheter, and spacings between the plurality of sub-needles and the plurality of supports are identical.

In a feasible solution, the electrode tube of the radio-frequency ablation catheter further includes a plurality of metal balls disposed at ends of the plurality of sub-needles, wherein
a ratio of an outer diameter of the metal balls to a diameter of the sub-needles is 1.05:1.01, and the plurality of metal balls are electrically conducted to the plurality of sub-needles.

In a feasible solution, the radio-frequency ablation catheter includes a fixing ring, wherein
the fixing ring is positioned within the puncture tube, and the fixing ring is configured to fix the plurality of supports and the plurality of sub-needles.

In a feasible solution, the fixing ring of the radio-frequency ablation catheter is provided with a plurality of through holes, the number of the plurality of through holes is equal to the sum of the number of the plurality of supports and the number of the plurality of sub-needles, and the plurality of supports and the plurality of sub-needles are disposed on the plurality of through holes in a penetrating fashion.

In a feasible solution, surfaces of the electrode tube and the signal conduit of the radio-frequency ablation catheter are provided with insulating layers, and the insulating layers are configured to shield a signal.

In a feasible solution, the temperature sensors of the radio-frequency ablation catheter may be thermistors.

In a feasible solution, the plurality of sub-needles of the radio-frequency ablation catheter are of a flower radial shape, and the plurality of supports are of a flower radial shape.

In a feasible solution, the lengths of the plurality of sub-needles of the radio-frequency ablation catheter are the longest in the middle parts and the shortest at the outermost ends, and are gradually shortened from the middle parts towards two ends.

In a feasible solution, the plurality of sub-needles of the radio-frequency ablation catheter are distributed symmetrically with respect to a central axis of the electrode tube, and the lengths of the corresponding sub-needles symmetrical with respect to the central axis of the electrode tube are identical.

In a feasible solution, the lengths of the plurality of supports of the radio-frequency ablation catheter are the longest in the middle parts and the shortest at the outmost ends, and the length isare gradually shortened from the middle parts towards two ends.

In a feasible solution, the plurality of supports of the radio-frequency ablation catheter are distributed symmetrically with respect to a central axis of the signal conduit, and the lengths of the corresponding supports symmetrical with respect to the central axis of the signal conduit are identical.

In a feasible solution, the plurality of sub-needles of the radio-frequency ablation catheter are distributed in a spherical space in a surrounding fashion, and the plurality of supports are distributed in a spherical space in a surrounding fashion.

In a feasible solution, each of the metal balls of the radio-frequency ablation catheter has the same latitude relative to the spherical space.

In a feasible solution, each of the temperature sensors of the radio-frequency ablation catheter has the same latitude relative to the spherical space.

In a feasible solution, each of the temperature sensors and each of the metal balls of the radio-frequency ablation catheter have the same latitude.

The present disclosure further provides a radio-frequency ablation system, including a radio-frequency ablation catheter in any of the above feasible solutions.

As can be seen from the above solutions, the radio-frequency ablation catheter of the present disclosure includes the needle tube portion and the handle portion. The sleeve on the handle portion is sleeved on the booster of the handle portion. One end of the booster is provided with the conductive joint, and the conductive joint is used for the external radio-frequency ablation system. The puncture tube on the needle tube portion is fixed at one end of the sleeve, the electrode tube and the signal conduit of the needle tube portion are fixed on the conductive joint, and the signal conduit is positioned on one side of the electrode tube. The plurality of sub-needles are disposed at the end of the electrode tube, and the plurality of supports are disposed at the end of the signal conduit. The capacitance thermometer is correspondingly disposed on each support. In the radio-frequency ablation catheter of the present disclosure, the signal conduit and the electrode tube are fixed on the conductive joint, the plurality of supports are fixed on one sides of the plurality of sub-needles, and the corresponding capacitance thermometers are disposed on the plurality of supports and configured to detect a change in the temperatures near the sub-needles. The radio-frequency ablation system releases the current into the human tissue through the electrode tube and the sub-needles, and makes a large number of dielectrics such as ions, water and colloidal particles in a human body fluid move at a high speed with the current under the action of a radio-frequency current vibrating at high frequency. Due to differences in sizes, mass charges and moving speeds of the ions, the tissue generates a biological heat effect due to friction of the ions, and thus the local temperature of the tissue rises. The capacitance thermometers on the supports sense the temperatures near the corresponding sub-needles and transmit the temperatures to the radio-frequency ablation system through the signal conduit. The change of the temperature within the human tissue maybe intuitively displayed on the radio-frequency ablation system. According to the temperature change range measured by the capacitance thermometer, an output current of the radio-frequency ablation system may be controlled. As a result, the controllability of the current in the surgery may be realized.

### BRIEF DESCRIPTION OF THE DRAWINGS

In order to explain embodiments of the present disclosure or technical solutions of the present disclosure more clearly, accompanying drawings required to be used in describing the embodiments or the prior art will be briefly introduced below. Obviously, the accompanying drawings in the following description are only some embodiments of the present disclosure. For those ordinarily skilled in the art, other accompanying drawings may be obtained based on these accompanying drawings without any creative labor.
FIG. 1 is a schematic diagram showing a structure of a radio-frequency ablation catheter according to a first embodiment of the present disclosure;
FIG. 2 is a partially enlarged schematic diagram showing a needle tube portion according to the first embodiment of the present disclosure;
FIG. 3 is a schematic diagram showing a structure of a radio-frequency ablation catheter according to a third embodiment of the present disclosure;
FIG. 4 is a partially enlarged schematic diagram showing a needle tube portion according to the third embodiment of the present disclosure;
FIG. 5 is a schematic diagram showing a structure of a radio-frequency ablation catheter according to a fourth embodiment of the present disclosure;
FIG. 6 is a partially enlarged schematic diagram showing a needle tube portion according to the fourth embodiment of the present disclosure;
FIG. 7 is a diagram of a withdrawn state of a booster according to the first embodiment of the present disclosure;
FIG. 8 is a perspective view showing sub-needles and supports according to the fourth embodiment of the present disclosure; and
FIG. 9 is a schematic diagram showing sub-needles and supports at the same latitude according to a fifth embodiment of the present disclosure.

### Symbols in the figures:

1: needle tube portion; 11: puncture tube; 12: electrode tube; 121: sub-needle; 122: metal ball; 13: signal conduit; 131: support; 132: temperature sensor; 2: handle portion; 21: sleeve; 22: booster; 221: conductive joint; 3: fixing ring; 31: through hole; and 4: insulating layer.

### DESCRIPTION OF THE EMBODIMENTS

In order to make objectives, technical solutions, and advantages of embodiments of the present disclosure clearer, the technical solutions in the embodiments of the present disclosure will be further described in detail below with reference to accompanying drawings in the embodiments of the present disclosure. Obviously, the described embodiments are only some, but not all of the embodiments of the present disclosure. Based on the embodiments of the present disclosure, all other embodiments obtained by those ordinarily skilled in the art without any creative labor shall fall within a protective scope of the present disclosure.

In the description of the present disclosure, it should be understood that orientations or position relationships indicated by terms "center", "longitudinal", "transverse", "upper", "lower", "front", "rear", "left", "right", "vertical", "horizontal", "top", "bottom", "inner", "outer", "axial", "radial" and "circumferential" are based on orientations or position relationships shown in the accompanying drawings, only for the convenience of describing the present disclosure and simplifying the description, rather than indicating or implying that a device or element in question must have a specific orientation and is constructed and operated in a specific orientation, such that they may not be understood as a limitation of the present disclosure.

In the present disclosure, unless otherwise specified and limited, terms "install", "connect with", "connect", "fix" and the like shall be understood in a broad sense, for example, they may be fixed connection, removable connection or integrated; or they may be mechanical connection, electrical connection or communication connection; or they may be direct connection or indirect connection through an intervening medium; or they may be communication inside two elements or interaction relationship between two elements, unless otherwise clearly defined. For those skilled in the art, specific meanings of the above terms in the present disclosure may be understood according to specific situations. The technical solutions of the present disclosure will described in detail below in combination with specific embodiments. The following specific embodiments may be combined with each other, and the same or similar concepts or processes may be omitted in some embodiments.

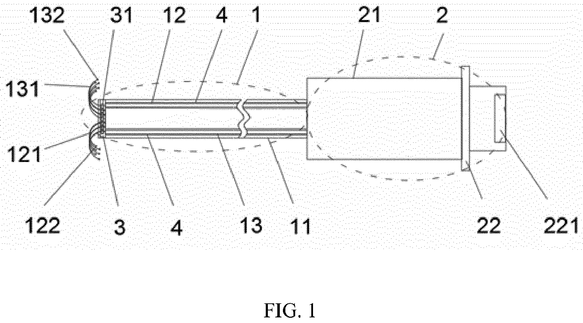

FIG. 1 is a schematic diagram showing a structure of a radio-frequency ablation catheter according to a first embodiment of the present disclosure.

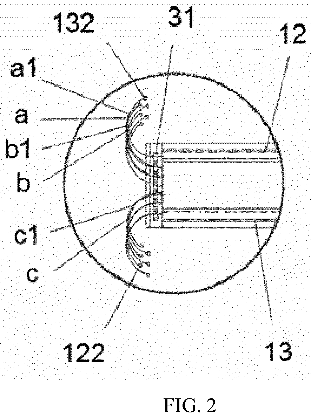

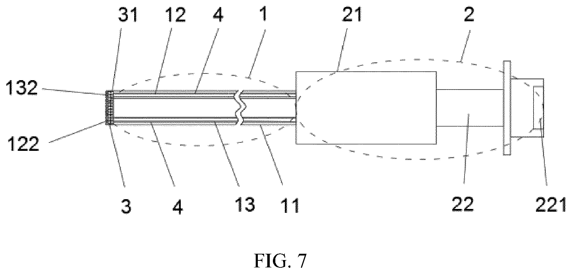

The radio-frequency ablation catheter in this embodiment is applied to a radio-frequency ablation system. The radio-frequency ablation catheter includes a needle tube portion 1 and a handle portion 2. The handle portion 2 includes a sleeve 21 and a booster 22. The sleeve 21 is sleeved on one end of the booster 22, the booster 22 may slide inside the sleeve 21, and a conductive joint 221 is provided at the other end of the booster 22. In this embodiment, four electrode jacks (not illustrated) are provided on the conductive joint 221, and configured to insert four electrode pins on a socket of the radio-frequency ablation system. The needle tube portion 1 includes a puncture tube 11, an electrode tube 12 and a signal conduit 13. Both the electrode tube 12 and the signal conduit 13 are disposed inside the puncture tube 11. The signal conduit 13 is positioned on one side of the puncture tube 11 and may move in the puncture tube 11. One end of the puncture tube 11 is fixed to an end of the sleeve 21, one ends of the electrode tube 12 and the signal conduit 13 are fixed on the conductive joint 221, and the electrode tube 12 and the signal conduit 13 are disposed inside the sleeve 21 in a penetrating fashion. When the booster 22 is pushed into the sleeve 21, the electrode tube 12 and the signal conduit 13 may be withdrawn from the puncture tube 11 by means of an action of the booster 22 (as shown in FIG. 7, which is a diagram of a withdrawn state of an electrode tube and a signal conduit from a puncture tube). When the electrode tube and the signal conduit are withdrawn from the puncture tube, the metal balls and the temperature sensors are arranged in a scattered and staggered fashion, and thus the friction between the metal balls and the temperature sensors in the stowed state may be reduced. A plurality of sub-needles 121 are disposed on the electrode tube 12, and a plurality of supports 131 are disposed on the signal conduit 13, wherein the number of the supports 131 is equal to that of the sub-needles 121. The sub-needles 121 on the electrode tube 12 are released in an umbrella shape (see FIG. 2), and the supports 131 are released in an umbrella shape. A current on the radio-frequency ablation system is transferred into a human tissue through the conductive joint 221, the electrode tube 12 and the sub-needles 121. A temperature sensor 132 is fixed at an end of each support 131. The temperature sensor 132 may be a capacitance thermometer. With a capacitor as a sensing element, a conversion device which converts the measured temperature into a capacitance change is formed as a capacitor with variable parameters. As the temperature rises, capacitance characteristics decrease gradually. A change in the capacitance characteristics is fed back to the radio-frequency ablation system, and a temperature change range may be seen institutively. A large number of dielectrics such as ions, water and colloidal particles in a human body fluid move at a high speed with the current under the action of a radio-frequency current vibrating at high frequency transmitted from the sub-needles 121. Due to differences in sizes, mass charges and moving speeds of the ions, the tissue generates a biological heat effect due to friction of the ions, and thus the local temperature of the tissue rises. However, the capacitance thermometers on the supports 131 next to the sub-needles 121 may be configured to detect a temperature inside the tissue. Each capacitance thermometer transmits the detected temperature value to the radio-frequency ablation system through the signal conduit 13, converts the sensed temperature into a displayable output signal, and intuitively exhibits a temperature range.

As may be seen from the above contents that the radio-frequency ablation catheter of the present disclosure includes the needle tube portion 1 and the handle portion 2. The sleeve 21 on the handle portion 2 is sleeved on the booster 22 of the handle portion 2. One end of the booster 22 is provided with the conductive joint 221, and the conductive joint 221 is used for the external radio-frequency ablation system. The puncture tube 11 on the needle tube portion 1 is fixed to one end of the sleeve 21, the electrode tube 12 and the signal conduit 13 of the needle tube portion 1 are fixed on the conductive joint 221, and the signal conduit 13 is positioned on one side of the electrode tube 12. The plurality of sub-needles 121 are disposed at the end of the electrode tube 12, and the plurality of supports 131 are disposed at the end of the signal conduit 13. The capacitance thermometer is correspondingly disposed on each support 131, and the signal conduit, the supports and the capacitance thermometers are electrically conducted. In the radio-frequency ablation catheter of the present disclosure, the signal conduit 13 and the electrode tube 12 are fixed on the conductive joint 221, the plurality of supports 131 are fixed on one sides of the plurality of sub-needles 121, and the corresponding capacitance thermometers are disposed on the plurality of supports 131 and configured to detect a change in temperatures near the sub-needles 121. The radio-frequency ablation system releases the current into the human tissue through the electrode tube 12 and the sub-needles 121, and makes a large number of dielectrics such as ions, water and colloidal particles in a human body fluid move at a high speed with the current under the action of the radio-frequency current vibrating at high frequency. Due to differences in sizes, mass charges and moving speeds of the ions, the tissue generates a biological heat effect due to friction of the ions, and thus the local temperature of the tissue rises. However, the capacitance thermometers on the supports 131 sense temperatures next to the corresponding sub-needles 121, and transmit the temperatures to the radio-frequency ablation system through the signal conduit 13, such that the temperature in the human tissue may be intuitively displayed on the radio-frequency ablation system. According to the temperature change range measured by the capacitance thermometers, the output current of the radio-frequency ablation system may be controlled, such that the controllability of the current in the surgery may be realized.

As shown in FIG. 2, optionally, in this embodiment, a plurality of sub-needles 121 are disposed on the electrode tube 12, a plurality of supports 131 are disposed on the signal conduit 13, and the signal conduit 13 is fixed on one side of the electrode tube 12. The number of the sub-needles 121 is equal to that of the supports 131. A support 131 is disposed next to each sub-needle 121, and distances and angles between each sub-needle 121 and each support 131 are identical. For example, a corresponding support a1 is disposed next to the sub-needle a, a corresponding support b1 is disposed next to the sub-needle b, a corresponding support c1 is disposed next to the sub-needle c, each sub-needle 121 has a corresponding support 131, a distance between the sub-needle a and an end of the support c1 is 0.2cm, a distance between an end of the sub-needle b and an end of the support b1 is 0.2cm, a distance between an end of the sub-needle c and an end of the support c1 is 0.2cm, distances between the sub-needle a and the sub-needle b and between the sub-needle b and the sub-needle c are both 0.4cm, and distances between the support a1 and the support b1 and between the support b1 and the support c1 are both 0.4cm as well. A distance between each sub-needle 121 is fixed, and a distance between each support 131 is fixed.

Optionally, in this embodiment, a metal ball 122 is welded to an end of each sub-needle 121, and a ratio of an outer diameter of each metal ball 122 to a diameter of each sub-needle 121 is 1.05:1.01. The outer diameter of the metal ball 122 is slightly greater than the diameter of the sub-needle 121, and the metal balls are staggered within the puncture tube, such that the metal balls may be well retracted into the puncture tube. The metal balls, the sub-needles and the electrode tube are electrically conducted. The surface area is spherical, which is greater than the interface area between the sub-needle 121 and the human tissue, such that the current release area may be increased. Accordingly, more tissue cells are in contact with the current, and the working efficiency of the radio-frequency ablation catheter is increased.

Optionally, in this embodiment, the radio-frequency ablation catheter further includes a fixing ring 3. The fixing ring 3 is clamped at a position not far from an opening of the puncture tube 11. The fixing ring 3 is a circular ring. A plurality of through holes 31 are provided in the circular ring, and the number of these through holes 31 is equal to the sum of the number of sub-needles 121 and the number of the supports 131. The diameter of the through holes 31 is greater than that of the sub-needles 121 and the supports 131. The plurality of sub-needles 121 and supports 131 are disposed on the through holes 31 in a penetrating fashion, and the diameter of the metal balls 122 and the capacitance thermometers is greater than that of the through holes 31 to facilitate the extension of the electrode tube 12 and the signal conduit 13, and the positions of the plurality of supports 131 and the plurality of sub-needles 121 may be fixed.

Optionally, in this embodiment, outer walls of the electrode tube 12 and the signal conduit 13 are wrapped with insulating layers 4. The insulating layers 4 are made of plastic. Due to the current passing through the electrode tube 12, the signal conduit 13 converts the temperature into an output signal. As a result, in order to prevent interference between the electrode tube 12 and the signal conduit 13, the plastic on the outer walls of the electrode tube 12 and the signal conduit 13 may prevent signal interference therebetween so as to achieve the function of shielding a signal.

A second embodiment is an alternative of the first embodiment, with a difference in that the capacitance thermometer in the first embodiment is replaced with a thermistor. The thermistor is sensitive to a temperature and shows different resistance values at different temperatures. The higher the temperature is, the lower the resistance value is. Under the action of the current, ions and media within the human tissue will operate at high speed and locally generate heat. When the temperature is higher and higher, the resistance within a local range of the thermistor will decrease with the increase of the temperature. The thermistors, the supports and the signal conduit are electrically conducted. When the resistance values of the thermistors change, the change in the resistance values is transmitted to the radio-frequency ablation system by means of the signal conduit 13, and a change range of a local temperature of the resistance value is calculated according to the change in the resistance value on the radio-frequency ablation system, such that the function of controlling the temperature may be achieved by controlling the output current of the radio-frequency ablation system.

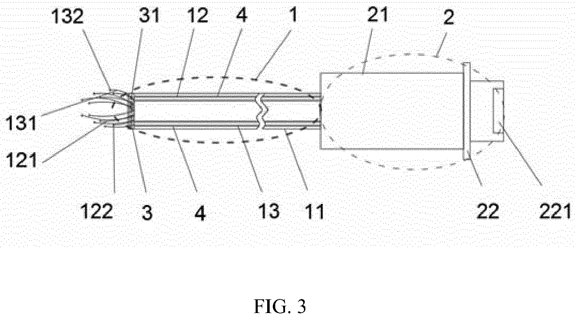

FIG. 3 is a schematic diagram showing a structure of a radio-frequency ablation catheter according to a third embodiment of the present disclosure.

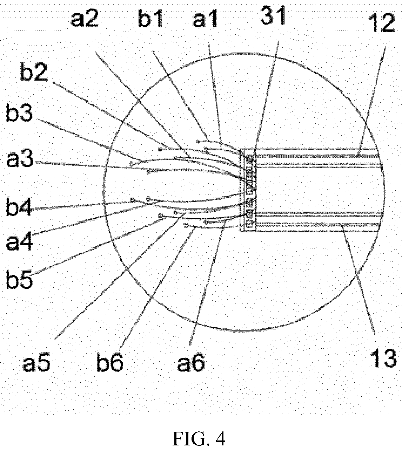

As described in FIG. 3, the third embodiment is an improved solution of the first embodiment, with an improvement in that a plurality of sub-needles 121 and a plurality of supports 131 are both of a flower radial shape (see FIG. 4). The lengths of the plurality of sub-needles 121 are different, and the positions of the plurality of sub-needles 121 are distributed symmetrically with respect to a central axis of the electrode tube 12. It is assumed that six sub-needles 121 are disposed on the electrode tube 12, and named as a1, a2, a3, a4, a5 and a6 respectively, wherein the a1 and the a6 are symmetrical with respect to the central axis of the electrode tube 12, the a2 and the a5 are symmetrical with respect to the central axis of the electrode tube 12, and the a3 and the a4 are symmetrical with respect to the central axis of the electrode tube 12. Moreover, the lengths of the a1 and the a6 are identical, the lengths of the a2 and the a5 are identical, and the lengths of a3 and a4 are identical, wherein the a1 and the a6 are positioned on outermost sides of all sub-needles 121 and have the shortest length, followed by the a2 and the a5, and the length of the a2 and the a5 is greater than the length of the a1 and the a6. The longest sub-needles are the a3 and the a4 which are positioned in the middlemost. According to such a position arrangement, different position of each sub-needle 121 makes the current flow through different positions. As a result, a current flow range may be enlarged and more human tissues are promoted to generate heat. The plurality of supports 131 have different lengths, and the positions of the plurality of supports 131 are distributed symmetrically with respect to the central axis of the signal conduit 13. It is assumed that six supports 131 are disposed on the signal conduit 13, and are named as b1, b2, b3, b4, b5 and b6 respectively, wherein the b1 and the b6 are symmetrical with respect to the central axis of the signal conduit 13, the b2 and the b5 are symmetrical with respect to the central axis of the signal conduit 13, and the b3 and the b4 are symmetrical with respect to the central axis of the signal conduit 13. Moreover, the lengths of the b1 and the b6 are identical, the lengths of the b2 and the b5 are identical, and the lengths of the b3 and the b4 are identical, wherein the b1 and the b6 are positioned on outermost sides of all supports 131 and have the longest length, followed by the b2 and the b5, and the length of the b2 and the b5 is greater than the length of the b1 and the b6. The longest sub-needles are the b3 and the b4, and the b3 and the b4 are positioned in the middlemost. The positions of the supports 131 are set according to the positions of the sub-needles 121. Each support 131 corresponds to a sub-needle 121. In this way, the temperature value generated near the corresponding sub-needle 121 may be specifically measured by the capacitance thermometer on the support 131. According to the difference in the heat generated by each sub-needle 121, the temperature measured by the capacitance thermometer is different as well, the resulting temperature is more targeted, and the resulting feedback data will be different as well. These temperature values are transmitted to the radio-frequency ablation system by means of the signal conduit 13. Accordingly, a specific temperature range may be obtained.

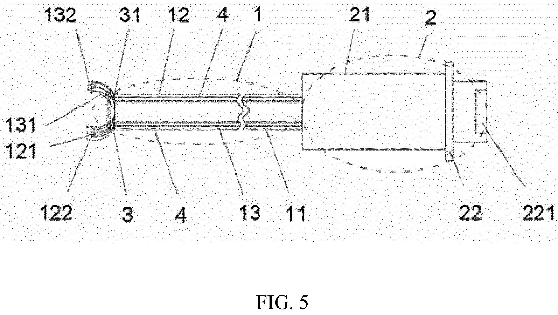

FIG. 5 is a schematic diagram showing a structure of a radio-frequency ablation catheter according to a fourth embodiment of the present disclosure.

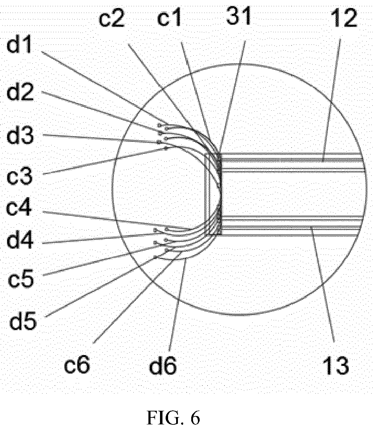

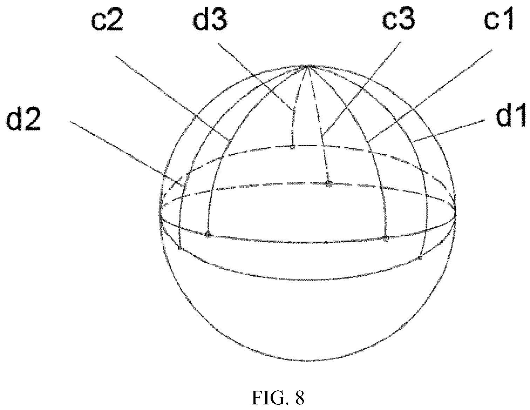

The fourth embodiment shown in FIG. 5 is an improved solution of the first embodiment, with an improvement in that a plurality of sub-needles 121 and a plurality of supports 131 are distributed in a spherical space. As shown in FIG. 6, the plurality of sub-needles 121 may be named as c1, c2, c3, c4, c5 and c6 respectively, wherein the c1, the c2, the c3, the c4, the c5 and the c6 are sequentially arranged from left to right, and have the same length. Since the six sub-needles 121 are distributed in the spherical space, although the six sub-needles 121 are in different horizontal positions, according to the spherical space, the corresponding metal balls 122 on the six sub-needles 121 have a wider current radiation range from left to right and a larger transverse distribution area at the same latitude, which facilitates contact of more lateral tissue cells. The plurality of supports 131 may be named as d1, d2, d3, d4, d5 and d6 respectively, wherein the d1, the d2, the d3, the d4, the d5 and the d6 are sequentially arranged from left to right. Each support 131 corresponds to a sub-needle 121, a capacitance thermometer on each support 131 is configured to detect a temperature near the corresponding metal ball 122, and the d1, the d2, the d3, the d4, the d5 and the d6 have the same length. According to a distribution diagram of the spherical space as shown in FIG. 8, since the six supports 131 are distributed in the spherical space, although the capacitance thermometers on the d1, the d2, the d3, the d4, the d5 and the d6 are at different horizontal positions in terms of horizontal positions, according to the spherical space, the corresponding capacitance thermometers on the six supports 131 are at the same latitude, and each capacitance thermometer transmits the detected temperature near the corresponding metal ball 122 to the radio-frequency ablation system by means of the signal conduit 13. As a result, the corresponding temperature change range may be seen intuitively on the radio-frequency ablation system. Further, the output current of the radio-frequency ablation system may be adjusted according to the change in the temperature.

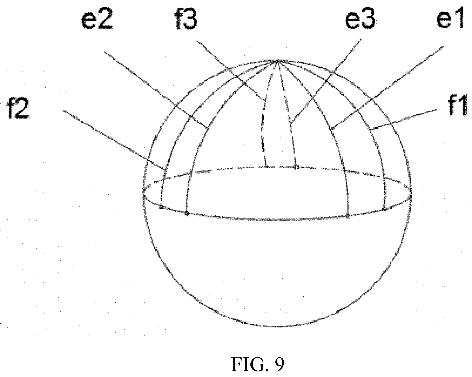

FIG. 9 is a schematic diagram showing structures of sub-needles and supports at the same latitude according to a fifth embodiment of the present disclosure.

The fifth embodiment as shown in FIG. 9 is an improved solution of the first embodiment, with an improvement in that a plurality of sub-needles 121 and a plurality of supports 131 are distributed in a spherical shape. The plurality of sub-needles may be named as e1, e2 and e3 respectively, wherein the e1, the e2 and the e3 have the same length. Since the three sub-needles 121 are distributed in the spherical space, although the three sub-needles 121 are in different horizontal positions, according to the spherical space, the corresponding metal balls 122 on the three sub-needles 121 are at the same latitude. The plurality of supports 131 may be named as f1, f2 and f3 respectively. Each support 131 corresponds to a sub pin 121. The capacitance thermometer on each support 131 is configured to detect a temperature near the corresponding metal ball 122, and the f1, the f2 and the f3 have the same length. According to the distribution of the spherical space as shown in FIG. 9, since the three supports 131 are distributed in the spherical space, although the capacitance thermometers on the f1, the f2 and the f3 are in different horizontal positions in terms of horizontal positions, according to the spherical space, the corresponding capacitance thermometers on the three supports 131 are at the same latitude. For this embodiment, the metal balls and the capacitance thermometers are at the same latitude in the spherical space.

In the present disclosure, unless otherwise expressly specified and limited, a first feature "on" or "under" a second feature may mean that the first feature is in direct contact with the second feature, or the first feature is in indirect contact with the second feature through an intervening medium.

Moreover, the first feature "on", "above" and "over" the second feature may mean that the first feature is directly above or obliquely above the second feature, or only means that a horizontal height of the first feature is higher than that of the second feature. The first feature "under", "below" and "underneath" the second feature may mean that the first feature is directly below or obliquely below the second feature, or only means that the horizontal height of the first feature is lower than that of the second feature.

In the description of this specification, reference is made to descriptions of terms "one embodiment", "some embodiments", "examples", "specific examples" or "some examples", which means that specific features, structures, materials or characteristics described in combination with embodiments or examples are included in at least one embodiment or example of the present disclosure. In this specification, schematic expressions of the above terms do not have to be for the same embodiments or examples. Further, the specific features, structures, materials or characteristics described may be combined in a suitable fashion in any one or more embodiments or examples. In addition, those skilled in the art may incorporate and combine different embodiments or examples described in this specification and features of different embodiments or examples, without mutual contradiction.

Finally, it should be noted that the above embodiments are only used to explain the technical solution of the present disclosure and shall not be construed as limitation. Although the present disclosure has been described in detail with reference to the above embodiments, those skilled in the art should understand that it may still modify the technical solutions recorded in the above embodiments or substitute some or all of the technical features equally, without making the essence of the corresponding technical solution deviate from the scope of the technical solution of each embodiment of the present disclosure.

## Claims

1. A radio-frequency ablation catheter applied to a radio-frequency ablation system, comprising:
a needle tube portion (1) and a handle portion (2), wherein
the handle portion (2) comprises a sleeve (21) and a booster (22), wherein
the sleeve (21) is sleeved on the booster (22),
the booster (22) is slidably arranged at one end of the sleeve (21),
the booster (22) is provided with a conductive joint (221), and
the conductive joint (221) is used for an external radio-frequency ablation system;
the needle tube portion (1) comprises a puncture tube (11), an electrode tube (12) and a signal conduit (13), wherein
the puncture tube (11) is fixed at the other end of the sleeve (21),
the electrode tube (12) is slidably disposed within the puncture tube (11), one end of the electrode tube (12) is fixed on the conductive joint (221) and the other end of the electrode tube (12) is provided with a plurality of sub-needles (121),
the plurality of sub-needles (121) are configured to transfer a current provided by the conductive joint (221),
the signal conduit (13) is slidably disposed in the puncture tube (11),
the signal conduit (13) is positioned on one side of the electrode tube (12), and
one end of the signal conduit (13) is fixed on the conductive joint (221);
the signal conduit (13) comprises a plurality of supports (131) and a plurality of temperature sensors (132);
the other end of the signal conduit (13) is provided with the plurality of supports (131), and the plurality of supports (131) are positioned on one sides of the plurality of sub-needles (121); and
the plurality of temperature sensors (132) are disposed on the plurality of supports (131) and are electrically conducted to the plurality of supports (131), and
the plurality of temperature sensors (132) are configured to detect temperatures near the plurality of sub-needles (121) and transmit them to the radio-frequency ablation system by means of the signal conduit (13),
**characterized in that**:
the radio-frequency ablation catheter further comprises a fixing ring (3) clamped within the puncture tube (11) at a position adjacent to an opening of the puncture tube (11), wherein
the fixing ring (3) is provided with a plurality of through holes (31), and the plurality of supports (131) and the plurality of sub-needles (121) extend through the plurality of through holes (31) respectively, and wherein
the plurality of sub-needles (121) are of a flower radial shape, and the plurality of supports (131) are of a flower radial shape.

2. The radio-frequency ablation catheter according to claim 1, wherein
the plurality of temperature sensors (132) are positioned at ends of the plurality of supports (131) and/or the temperature sensors (132) are thermistors.

3. The radio-frequency ablation catheter according to claim 1, wherein
the temperature sensors (132) are capacitance thermometers.

4. The radio-frequency ablation catheter according to claim 1, wherein
the plurality of supports (131) and the plurality of sub-needles (121) are identical in numbers and are arranged alternately.

5. The radio-frequency ablation catheter according to claim 1, wherein
the plurality of supports (131) are disposed next to the plurality of sub-needles (121), and spacings between the plurality of sub-needles (121) and the plurality of supports (131) are identical.

6. The radio-frequency ablation catheter according to claim 1, wherein
the electrode tube (12) further comprises a plurality of metal balls (122) disposed at ends of the plurality of sub-needles (121), wherein
a ratio of an outer diameter of the metal balls (122) to a diameter of the sub-needles (121) is 1.05:1.01, and the plurality of metal balls (122) are electrically conducted to the plurality of sub-needles (121), and wherein
the diameter of the metal ball (122) and the diameter of the temperature sensors (132) are both greater than that of the through hole (31).

7. The radio-frequency ablation catheter according to claim 1, wherein the fixing ring is a circular ring ,
the number of the plurality of through holes (31) is equal to the sum of the number of the plurality of supports (131) and the number of the plurality of sub-needles (121), .

8. The radio-frequency ablation catheter according to claim 1, wherein
surfaces of the electrode tube (12) and the signal conduit (13) are provided with insulating layers (4), and the insulating layers (4) are configured to shield a signal.

9. The radio-frequency ablation catheter according to claim 1, wherein
each of the plurality of sub-needle (121) and the plurality of supports (131) is convex radially outwardly with respect to a line connecting a proximal end adjacent to the fixing ring (3) and a distal end away from the fixing ring (3).

10. The radio-frequency ablation catheter according to claim 9, wherein
the lengths of the plurality of sub-needles (121) are the longest in the middle parts and the shortest at the outermost ends, and are gradually shortened from the middle part towards two ends.

11. The radio-frequency ablation catheter according to claim 10, wherein
the plurality of sub-needles (121) are distributed symmetrically with respect to a central axis of the electrode tube (12), and
the lengths of the corresponding sub-needles (121) symmetrical with respect to the central axis of the electrode tube (12) are identical.

12. The radio-frequency ablation catheter according to claim 9, wherein
the lengths of the plurality of supports (131) are the longest in the middle parts and the shortest at the outmost ends, and are gradually shortened from the middle parts towards two ends, optionally wherein
the plurality of supports (131) are distributed symmetrically with respect to a central axis of the signal conduit (13), and the lengths of the corresponding supports (131) symmetrical with respect to the central axis of the signal conduit (13) are identical.

13. The radio-frequency ablation catheter according to claim 6, wherein
the plurality of sub-needles (121) are distributed in a spherical space in a surrounding fashion, and
the plurality of supports (131) are distributed in a spherical space in a surrounding fashion.

14. The radio-frequency ablation catheter according to claim 6, wherein
a) each of the metal balls (122) has the same latitude relative to the spherical space;
b) each of the temperature sensors (132) has the same latitude relative to the spherical space; or
c) the temperature sensors (132) and the metal balls (122) have the same latitude.

15. A radio-frequency ablation system, comprising:
a radio-frequency ablation catheter according to any one of claims 1 to 14.

## Patentansprüche

1. Hochfrequenz-Ablationskatheter zur Anwendung bei einem Hochfrequenz-Ablationssystem, umfassend:
einen Nadelrohrabschnitt (1) und einen Griffabschnitt (2), wobei
der Griffabschnitt (2) eine Hülse (21) und ein Vorschubelement (22) umfasst, wobei
die Hülse (21) auf das Vorschubelement (22) aufgeschoben ist,
das Vorschubelement (22) verschiebbar an einem Ende der Hülse (21) angeordnet ist,
das Vorschubelement (22) mit einem leitfähigen Anschluss (221) versehen ist, und
der leitfähige Anschluss (221) für ein externes Hochfrequenz-Ablationssystem vorgesehen ist;
der Nadelrohrabschnitt (1) ein Punktionsrohr (11), ein Elektrodenrohr (12) und eine Signalleitung (13) umfasst, wobei
das Punktionsrohr (11) an dem anderen Ende der Hülse (21) befestigt ist,
das Elektrodenrohr (12) verschiebbar innerhalb des Punktionsrohrs (11) angeordnet ist,
ein Ende des Elektrodenrohrs (12) an dem leitfähigen Anschluss (221) befestigt ist, und
das andere Ende des Elektrodenrohrs (12) mit einer Vielzahl von Teilnadeln (121) versehen ist,
die Vielzahl von Teilnadeln (121) dafür konfiguriert sind, einen durch den leitfähigen Anschluss (221) bereitgestellten Strom zu übertragen,
die Signalleitung (13) verschiebbar in dem Punktionsrohr (11) angeordnet ist,
die Signalleitung (13) auf einer Seite des Elektrodenrohrs (12) positioniert ist, und
ein Ende der Signalleitung (13) an dem leitfähigen Anschluss (221) befestigt ist;
die Signalleitung (13) eine Vielzahl von Trägern (131) und eine Vielzahl von Temperatursensoren (132) umfasst;
das andere Ende der Signalleitung (13) mit der Vielzahl von Trägern (131) versehen ist und die Vielzahl von Trägern (131) auf einer Seite der Vielzahl von Teilnadeln (121) positioniert sind; und
die Vielzahl von Temperatursensoren (132) auf der Vielzahl von Trägern (131) angeordnet sind und mit der Vielzahl von Trägern (131) in elektrisch leitender Verbindung stehen, und
die Vielzahl von Temperatursensoren (132) dafür konfiguriert sind, Temperaturen in der Nähe der Vielzahl von Teilnadeln (121) zu erfassen und mittels der Signalleitung (13) an das Hochfrequenz-Ablationssystem zu übertragen,
**dadurch gekennzeichnet, dass**:
der Hochfrequenz-Ablationskatheter ferner einen Befestigungsring (3) umfasst, der innerhalb des Punktionsrohrs (11) an einer Position angrenzend an eine Öffnung des Punktionsrohrs (11) eingespannt ist, wobei
der Befestigungsring (3) mit einer Vielzahl von Durchgangslöchern (31) versehen ist und sich die Vielzahl von Trägern (131) und die Vielzahl von Teilnadeln (121) jeweils durch die Vielzahl von Durchgangslöchern (31) erstrecken, und wobei
die Vielzahl von Teilnadeln (121) eine blütenartig radiale Form aufweisen und die Vielzahl von Trägern (131) eine blütenartig radiale Form aufweisen.

2. Hochfrequenz-Ablationskatheter nach Anspruch 1, wobei
die Vielzahl von Temperatursensoren (132) an Enden der Vielzahl von Trägern (131) positioniert sind und/oder die Temperatursensoren (132) Thermistoren sind.

3. Hochfrequenz-Ablationskatheter nach Anspruch 1, wobei
die Temperatursensoren (132) Kapazitätsthermometer sind.

4. Hochfrequenz-Ablationskatheter nach Anspruch 1, wobei
die Vielzahl von Trägern (131) und die Vielzahl von Teilnadeln (121) in ihrer Anzahl identisch sind und alternierend angeordnet sind.

5. Hochfrequenz-Ablationskatheter nach Anspruch 1, wobei
die Vielzahl von Trägern (131) neben der Vielzahl von Teilnadeln (121) angeordnet sind und Abstände zwischen der Vielzahl von Teilnadeln (121) und der Vielzahl von Trägern (131) identisch sind.

6. Hochfrequenz-Ablationskatheter nach Anspruch 1, wobei
das Elektrodenrohr (12) ferner eine Vielzahl von Metallkugeln (122) umfasst, die an Enden der Vielzahl von Teilnadeln (121) angeordnet sind, wobei
ein Verhältnis eines Außendurchmessers der Metallkugeln (122) zu einem Durchmesser der Teilnadeln (121) 1,05:1,01 beträgt und die Vielzahl von Metallkugeln (122) mit der Vielzahl von Teilnadeln (121) in elektrisch leitender Verbindung stehen, und wobei
der Durchmesser der Metallkugel (122) und der Durchmesser der Temperatursensoren (132) beide größer als derjenige der Durchgangslöcher (31) sind.

7. Hochfrequenz-Ablationskatheter nach Anspruch 1, wobei der Befestigungsring ein kreisförmiger Ring ist, und
die Anzahl der Vielzahl von Durchgangslöchern (31) gleich der Summe aus der Anzahl der Vielzahl von Trägern (131) und der Anzahl der Vielzahl von Teilnadeln (121) ist.

8. Hochfrequenz-Ablationskatheter nach Anspruch 1, wobei
Oberflächen des Elektrodenrohrs (12) und der Signalleitung (13) mit Isolierschichten (4) versehen sind und die Isolierschichten (4) dafür konfiguriert sind, ein Signal abzuschirmen.

9. Hochfrequenz-Ablationskatheter nach Anspruch 1, wobei
jede der Vielzahl von Teilnadeln (121) und der Vielzahl von Trägern (131) in Bezug auf eine Verbindungslinie zwischen einem proximalen Ende angrenzend an den Befestigungsring (3) und einem distalen Ende entfernt von dem Befestigungsring (3) radial nach außen konvex gewölbt ist.

10. Hochfrequenz-Ablationskatheter nach Anspruch 9, wobei
die Längen der Vielzahl von Teilnadeln (121) in den mittleren Abschnitten am längsten und an den äußersten Enden am kürzesten sind und sich von dem mittleren Abschnitt zu den zwei Enden hin allmählich verkürzen.

11. Hochfrequenz-Ablationskatheter nach Anspruch 10, wobei
die Vielzahl von Teilnadeln (121) in Bezug auf eine Mittelachse des Elektrodenrohrs (12) symmetrisch verteilt sind, und
die Längen von bezüglich der Mittelachse des Elektrodenrohrs (12) zueinander symmetrischen Teilnadeln (121) identisch sind.

12. Hochfrequenz-Ablationskatheter nach Anspruch 9, wobei
die Längen der Vielzahl von Trägern (131) in den mittleren Abschnitten am längsten und an den äußersten Enden am kürzesten sind und sich von den mittleren Abschnitten zu den zwei Enden hin allmählich verkürzen, wobei optional
die Vielzahl von Trägern (131) in Bezug auf eine Mittelachse der Signalleitung (13) symmetrisch verteilt sind und die Längen von bezüglich der Mittelachse der Signalleitung (13) zueinander symmetrischen Trägern (131) identisch sind.

13. Hochfrequenz-Ablationskatheter nach Anspruch 6, wobei
die Vielzahl von Teilnadeln (121) umgebend in einem kugelförmigen Raum verteilt sind, und
die Vielzahl von Trägern (131) umgebend in einem kugelförmigen Raum verteilt sind.

14. Hochfrequenz-Ablationskatheter nach Anspruch 6, wobei
a) jede der Metallkugeln (122) denselben Breitengrad bezüglich des kugelförmigen Raums aufweist;
b) jeder der Temperatursensoren (132) denselben Breitengrad bezüglich des kugelförmigen Raums aufweist; oder
c) die Temperatursensoren (132) und die Metallkugeln (122) denselben Breitengrad aufweisen.

15. Hochfrequenz-Ablationssystem, umfassend:
einen Hochfrequenz-Ablationskatheter nach einem der Ansprüche 1 bis 14.

## Revendications

1. Cathéter d'ablation par radiofréquence appliqué à un système d'ablation par radiofréquence, comprenant :
une partie de tube d'aiguille (1) et une partie de poignée (2), dans lequel
la partie de poignée (2) comprend un manchon (21) et un amplificateur (22), dans lequel
le manchon (21) est emmanché sur l'amplificateur (22),
l'amplificateur (22) est agencé de manière coulissante à une extrémité du manchon (21),
l'amplificateur (22) est pourvu d'un joint conducteur (221), et
le joint conducteur (221) est utilisé pour un système d'ablation par radiofréquence externe ;
la partie de tube d'aiguille (1) comprend un tube de ponction (11), un tube d'électrode (12) et un conduit de signal (13), dans lequel
le tube de ponction (11) est fixé à l'autre extrémité du manchon (21),
le tube d'électrode (12) est disposé de manière coulissante à l'intérieur du tube de ponction (11), une extrémité du tube d'électrode (12) est fixée sur le joint conducteur (221) et l'autre extrémité du tube d'électrode (12) est pourvue d'une pluralité de sous-aiguilles (121),
la pluralité de sous-aiguilles (121) sont configurées pour transférer un courant fourni par le joint conducteur (221),
le conduit de signal (13) est disposé de manière coulissante dans le tube de ponction (11),
le conduit de signal (13) est positionné sur un côté du tube d'électrode (12), et
une extrémité du conduit de signal (13) est fixée sur le joint conducteur (221) ;
le conduit de signal (13) comprend une pluralité de supports (131) et une pluralité de capteurs de température (132) ;
l'autre extrémité du conduit de signal (13) est pourvue de la pluralité de supports (131), et la pluralité de supports (131) sont positionnés sur un côté de la pluralité de sous-aiguilles (121) ; et
la pluralité de capteurs de température (132) sont disposés sur la pluralité de supports (131) et sont électriquement conduits vers la pluralité de supports (131), et
la pluralité de capteurs de température (132) sont configurés pour détecter des températures à proximité de la pluralité de sous-aiguilles (121) et les transmettre au système d'ablation par radiofréquence au moyen du conduit de signal (13),
**caractérisé en ce que** :
le cathéter d'ablation par radiofréquence comprend en outre une bague de fixation (3) serrée à l'intérieur du tube de ponction (11) à une position adjacente à une ouverture du tube de ponction (11), dans lequel
la bague de fixation (3) est pourvue d'une pluralité de trous traversants (31), et la pluralité de supports (131) et la pluralité de sous-aiguilles (121) s'étendent à travers la pluralité de trous traversants (31) respectivement, et dans lequel
la pluralité de sous-aiguilles (121) sont d'une forme radiale de fleur, et la pluralité de supports (131) sont d'une forme radiale de fleur.

2. Cathéter d'ablation par radiofréquence selon la revendication 1, dans lequel
la pluralité de capteurs de température (132) sont positionnés à des extrémités de la pluralité de supports (131) et/ou les capteurs de température (132) sont des thermistances.

3. Cathéter d'ablation par radiofréquence selon la revendication 1, dans lequel les capteurs de température (132) sont des thermomètres capacitifs.

4. Cathéter d'ablation par radiofréquence selon la revendication 1, dans lequel
la pluralité de supports (131) et la pluralité de sous-aiguilles (121) sont identiques en nombre et sont agencées en alternance.

5. Cathéter d'ablation par radiofréquence selon la revendication 1, dans lequel la pluralité de supports (131) sont disposés à côté de la pluralité de sous-aiguilles (121), et les espacements entre la pluralité de sous-aiguilles (121) et la pluralité de supports (131) sont identiques.

6. Cathéter d'ablation par radiofréquence selon la revendication 1, dans lequel
le tube d'électrode (12) comprend en outre une pluralité de billes métalliques (122) disposées aux extrémités de la pluralité de sous-aiguilles (121), dans lequel
un rapport d'un diamètre extérieur des billes métalliques (122) sur un diamètre des sous-aiguilles (121) est de 1,05:1,01, et la pluralité de billes métalliques (122) sont électriquement conduites vers la pluralité de sous-aiguilles (121), et dans lequel
le diamètre de la bille métallique (122) et le diamètre des capteurs de température (132) sont tous deux supérieurs à celui du trou traversant (31).

7. Cathéter d'ablation par radiofréquence selon la revendication 1, dans lequel la bague de fixation est une bague circulaire,
le nombre de la pluralité de trous traversants (31) est égal à la somme du nombre de la pluralité de supports (131) et du nombre de la pluralité de sous-aiguilles (121).

8. Cathéter d'ablation par radiofréquence selon la revendication 1, dans lequel
des surfaces du tube d'électrode (12) et du conduit de signal (13) sont pourvues de couches isolantes (4), et les couches isolantes (4) sont configurées pour protéger un signal.

9. Cathéter d'ablation par radiofréquence selon la revendication 1, dans lequel
chacune de la pluralité de sous-aiguilles (121) et de la pluralité de supports (131) est convexe radialement vers l'extérieur par rapport à une ligne reliant une extrémité proximale adjacente à la bague de fixation (3) et une extrémité distale éloignée de la bague de fixation (3).

10. Cathéter d'ablation par radiofréquence selon la revendication 9, dans lequel
les longueurs de la pluralité de sous-aiguilles (121) sont les plus longues dans les parties médianes et les plus courtes aux extrémités les plus extérieures, et sont progressivement raccourcies de la partie médiane vers deux extrémités.

11. Cathéter d'ablation par radiofréquence selon la revendication 10, dans lequel
la pluralité de sous-aiguilles (121) sont réparties symétriquement par rapport à un axe central du tube d'électrode (12), et
les longueurs des sous-aiguilles (121) correspondantes symétriques par rapport à l'axe central du tube d'électrode (12) sont identiques.

12. Cathéter d'ablation par radiofréquence selon la revendication 9, dans lequel
les longueurs de la pluralité de supports (131) sont les plus longues dans les parties médianes et les plus courtes aux extrémités les plus extérieures, et sont progressivement raccourcies des parties médianes vers deux extrémités, facultativement dans lequel
la pluralité de supports (131) sont réparties symétriquement par rapport à un axe central du conduit de signal (13), et les longueurs des supports (131) correspondants symétriques par rapport à l'axe central du conduit de signal (13) sont identiques.

13. Cathéter d'ablation par radiofréquence selon la revendication 6, dans lequel
la pluralité de sous-aiguilles (121) sont réparties dans un espace sphérique d'une manière environnante, et
la pluralité de supports (131) sont réparties dans un espace sphérique d'une manière environnante.

14. Cathéter d'ablation par radiofréquence selon la revendication 6, dans lequel
a) chacune des billes métalliques (122) a la même latitude par rapport à l'espace sphérique ;
b) chacun des capteurs de température (132) a la même latitude par rapport à l'espace sphérique ; ou
c) les capteurs de température (132) et les billes métalliques (122) ont la même latitude.

15. Système d'ablation par radiofréquence, comprenant :
un cathéter d'ablation par radiofréquence selon l'une quelconque des revendications 1 à 14.
